# DEMANDE DE BREVET EUROPEEN

(11) **EP 0 834 334 A1**
(43) Date de publication de la demande: **08.04.1998**
(21) Numéro de dépôt: 97420149.3
(22) Date de dépôt: 27.08.1997
(51) Int. Cl.: A61M 39/16, B65D 43/16

(54) **Etui de protection semi rigide pour usage médical**

(30) Priorité: 04.10.1996 FR 9612577
(71) Demandeur: Hugon, Roland, 43160 La Chaise Dieu (FR); Grivon, Josette, 43270 Allègre (FR)
(72) Inventeur: Grivon Josette, 43270 Allègre (FR)
(74) Mandataire: Schmitz, Jean-Marie

(57) **Abrégé**

La présente invention concerne un étui de protection constitué d'au moins deux coquilles (1, 2 ) habillées de mousses ( 20, 23 ) reliées et articulées l'une par rapport à l'autre par une charnière ( 3 ) , dont le système de fermeture est constitué de portions de cercle ( 14 ) portées par des saillies en relief( 13 ) se logeant sous des languettes ( 15 ) correspondantes et par un système de retenue du robinet constitué d'un cylindre ( 17 ) et d'échancrures ( 18 ) présentant un léger rétrécissement ( 19 ) dans leur partie supérieure.

## Description

La présente invention concerne un étui de protection pour des articles médicaux, notamment pour un robinet à usage médical, semi-rigide à fermetures intérieures pour éviter les infections nosocomiales et la prolifération des microbes, bactéries, virus, autour et dans les appareils ou instruments de régulation ou autres, utilisés plus spécialement en milieu hospitalier ; tels que rampes à un ou plusieurs robinets, robinets 3 voies, cathéters, connecteurs. Ces appareils sont placés dans un milieu aseptique afin d'éviter la propagation des microbes.

il est décrit dans le brevet FR 2 578 746 un boîtier rigide en matière plastique moulé par injection avec une épaisseur de un à plusieurs millimètres et des reliefs pleins de matière. Sa principale caractéristique réside dans le fait que la mousse qui l'habille intérieurement porte des rainures creuses et des évidements correspondant aux passages des tubulures raccordées à l'article à protéger. Ce boîtier a une structure rigide due à la matière le constituant, à son épaisseur et à sa forme de cuvette. Il est indéformable. Le verrouillage des deux coquilles du boîtier en position fermée s'effectue à l'aide de deux système à doigts pleins, d'une coquille pénétrant dans une fenêtre ouverte du rebord de l'autre coquille et disposés sur les deux cotés opposés parallèles du boîtier. L'ouverture du boîtier est difficile puisqu'il faut exercer une forte pression sur les deux cotés opposés pour faire ressortir chaque téton de la fenêtre correspondante et en même temps exercer une force pour séparer les deux pièces l'une de l'autre. La difficulté est accrue par le fait des petites dimensions, de la forte rigidité et de la prise réduite du boîtier.

Dans ce système de boîtier rigide d'une épaisseur de 1,5 millimètre environ, les doigts de verrouillage pénètrent dans des ouvertures débouchantes placées dans le rebord de la coquille opposée. Compte tenu du fait que le boîtier est constitué dans un matériau rigide et que cette rigidité est augmentée considérablement par les formes de cuvettes et de rebords de chaque coquille, il devient alors très difficile de pouvoir fermer le boîtier par encliquetage en exerçant une pression vers l'extérieur sur chaque coquille. L'ouverture devient très difficile sinon impossible car il est nécessaire par pression importante de déformer l'une ou les deux coquilles pour désenclancher les doigts de verrouillage des ouvertures ou alors il faut essayer de passer un ongle ou un objet pointu entre les deux coquilles afin de provoquer leur écartement pour désenclancher les doigts de verrouillage. Ce système de fermeture ne fonctionne que dans sa théorie.

D'autre part, le site à protéger ne possède aucun système permettant de le retenir de façon certaine dans le boîtier. Les mousses formant des empreintes pour le site ne servent qu'à faciliter la fermeture du boîtier ce qui apparaît clairement puisque dans la revendication 5 la mousse comporte des plots séparés par des fentes encore une fois pour asurer la fermeture du boîtier mais absolument pas pour maintenir le site dans le boîtier. Lors de l'ouverture du boîter, il est impossible que le site reste en place dans le boîtier d'autant plus que l'ouverture très difficile est brusque. Le site sort du boîtier, il peut provoquer un arrachement des systèmes de connexions, il est nécessaire de remettre en place le site dans le boîtier, de le maintenir pendant la fermeture ; toutes ces opérations vont à l'encontre du but recherché.

De plus, le poids de ce boîtier est important, il est multiplie par quatre par rapport à la présente invention. Lorsqu'il n'est pas posé sur le lit mais suspendu par la ou les tubulures son poids se fait sentir par la traction qu'il exerce par l'aiguille qui pénètre dans le corps.

Le boîtier de protection de robinet décrit dans le document FR 2 7 08 073 est à l'inverse du boîtier rigide décrit ci dessus. Il est constitué d'une mousse de polyéthylène souple pressée à chaud. Dans ce genre de boîtier, « il n'est pas souhaitable » de l'avis de son inventeur « d'utiliser des moyens d'encliquetage car la déformabilité élastique du matériau du boîtier couduirait à un décliquetage de ses moyens et à une ouverture du boîtier sous l'effet des compressions qu'il peut être amené à subir ». Pour cette raison, les fermetures sont constituées par des boutons pressions portés par des pattes en une seule pièce avec chaque coquille et en saillie sur les bords à réunir opposé à celui formant charnière.

La mise en oeuvre pour la fermeture des boutons pressions portés par les pattes demande une manipulation importante due aux nombreuses pressions avec les doigts qu'il faut exercer pour fermer le système. Lorsqu'on veut ouvrir le boîtier souple, il est nécessaire pour la meilleure commodité de placer un ou des doigts légèrement à l'intérieur des coquilles en exerçant des forces opposées importante qui provoquent une brusque ouverture du boîtier risquant de provoquer son déchirement et / ou l'éjection du robinet. Ce système ne permet pas une bonne hygiène car il nécessite d'importantes manipulations manuelles sur le système de fermeture voir même sur les lèvres du boîtier, entraînant de ce fait un risque important de propagation des microbes dans le boîtier. De plus les pièces en saillie augmente les dimensions du boîtier et par conséquent un plus grand volume de stockage est nécessaire.

Le brevet CH 634 902 concerne les raccords conique plus particulièrement pour les dialysés et que la partie circulaire reliant les deux cônes est maintenue par un clips. Ce système n'a pas les mêmes fonctions que la présente invention.

Dans le brevet GB 2 136 775 il est décrit un container cylindrique dont le rebord inférieur du couvercle comporte sur sa circonférence un enroulement sphérique et en regard sur le corps du container un autre enroulement sphérique pour assurer une fermeture circulaire extérieure au container. Le système de fermeture de la présente invention est très différent puisqu'il s'effectue par points et par l'intérieur du boîtier.

Le brevet US 3 811 563 décrit une boîte épaisse et rigide en matière plastique moulée destinée à recevoir la seconde clé d'un véhicule automobile, comportant un couvercle supportant une butée ouverte coopérant avec un cran porté par le coté du boîtier. Ces deux faces du cran sont inclinées, si bien que le couvercle se verrouille et se déverrouille automatiquement en rapprochant ou en éloignant le couvercle du boîtier. Un tel système d'ouverture automatique n'est pas suffisamment sûr pour l'application comme protection pour un article médical. Cette technique ne suggère absolument pas l'objet de l'invention, même combinée à l'enseignement du brevet FR 2 578 746.

Il existe aussi d'autres boîtiers rigides dont le système de fermeture est à encliquetage. Pour les fermer, il faut exercer une pression relativement importante sur chaque coquille, pour les ouvrir il faut aussi exercer une traction importante en tirant sur une pane dite femelle de la fermeture d'un accès mal aisé car placé très prés du corps du boîtier. Cette ouverture pouvant être brusque risque de faire éjecter le robinet ou la rampe. Le poids est un inconvénient supplémentaire à ces boîtiers rigides, pouvant causer des meurtrissures s'ils sont en contact avec le cours du malade.

Le problème que vise à résoudre l'invention est de fournir un boîtier suffisamment solide pour résister aux pressions externes et de protéger mécaniquement et contre les infections bactériologiques ou virales, un article médical, en fournissant un produit simple, léger, facile à ouvrir, à fermer et d'une fabrication aisée.

L'enseignement du brevet FR 2 578 746 préconise de faire une structure épaisse rectangulaire, le brevet FR 2 708 073 enseigne un boîtier en mousse avec système de fermeture à pressions et dissuade d'utiliser des moyens d'encliquetage pour la fermeture. Le brevet US 3 811 563 enseigne une boite à structure rectangulaire épaisse à ouverture ou fermeture automatique par simple soulèvement ou rabat du couvercle.

La solution retenue par l'invention va à l'encontre de ces enseignements.

L'étui selon l'invention se caractérise principalement par le fait que contrairement aux enseignements divulgués ; il est réalisé en matière plastique dure et rigide mais qui en raison de sa faible épaisseur de l'ordre de 0,4 millimètre pour un encombrement de 6 centimètres, est déformable élastiquement justement en raison de cette faible épaisseur. Les reliefs sont toujours en creux et l'épaisseur est sensiblement identique sur la totalité de l'étui puisqu'il est obtenu par thermoformage. D'une façon surprenante, cette déformabilité qui pourrait constituer un défaut n'est pas gênante puisque la présence de mousses à l'intérieure augmente la résistance contre les pressions extérieures et empêche l'introduction des agents infectieux sur l'article à protéger. La simplicité de l'étui formé d'une seule pièce par thermoformage y compris ses moyens d'encliquetage et de maintien en position fermée se révèlent malgré la faible épaisseur, d'une résistance surprenante à toute ouverture accidentelle, ce qui n'était pas une évidence. La faible quantité de matière nécessaire est aussi un avantage pour obtenir un étui très léger et d un prix de revient minimum. A titre de comparaison, le produit décrit dans le brevet FR 2 578 746 est cinq fois plus lourd que l'étui selon l'invention pour le même article enveloppé.

De façon surprenante, la présente invention va à l'encontre des systèmes de fermeture connus et vise à remédier à ses inconvénients. A cet effet, le boîtier comprend au moins 2 coquilles articulées par une charnière et dont les faces concaves viennent se refermer l'une contre l'autre, provoquant la jonction de leur lèvre supérieure. Dans les parois perpendiculaires à la base des coquilles sont pratiqués un ou des orifices permettant le passage des tubulures. Une mousse de protection occupe le volume intérieur des coquilles. Un support maintient fixe le robinet. Les deux coquilles sont retenues l'une à l'autre par un système d'encliquetage à bossage convexe pénétrant sous un logement creux concave. L'invention sera mieux comprise à l'aide des descriptions qui suivent et des dessins.

La figure 1 montre une vue de l'étui ouvert avec les mousses s'y logeant et le robinet.

La figure 2 montre l'étui avec le système de charnière et de fermeture.

La figure 3 montre une coupe du système de fermeture et de tenu de l'article.

La figure 4 montre une variante vue en coupe du système de fermeture.

la figure 5 montre une autre variante vue en coupe du système de fermeture.

L'étui selon l'invention est constitué de deux coquilles (1,2) l'une formant le couvercle (2), l'autre formant la base (1). Toutes deux sont creuses, avantageusement de forme cylindrique tronquée selon l'axe du cylindre obtenu par thermoformage et se développant sur un arc de cercle de ordre de 260°. Elles sont reliées entre elles par le coté tronqué selon une charnière (3). La section transversale de cette charnière est en forme de deux portions de cercles sur les trois quart de leur circonférence et reliées entre elles par une pliure en forme de «U» ouvert (25) vers l'extérieur. Chaque portion de cercle opposée à la pliure en «U» est reliée à une coquille.

L'élasticité de la matière et l'épaisseur de la charnière est telle qu'au repos en position ouverte, le couvercle (2) fasse un angle compris entre 60° et 150° avec la base (1), ce qui permet avantageusement de manipuler l'article protégé sans autre intervention sur le boîtier.

Les deux coquilles peuvent être de même profondeur, mais de préférence, la base (1) a une profondeur moins importante que la couvercle, d'une quantité de l'ordre de un cinquième.

Les aillés latéraux (6) de chaque coquille sont sensiblement perpendiculaires à leur fond, mais pour faciliter le démoulage, il font un angle compris entre 90° et 100°, avec un angle préférentiel de 95 avec le fond de la coquille.

Les différentes surfaces de l'étui se raccordent sous des arrondis extérieurs (7) de rayon de courbure supérieur à 1 millimètre pour éviter de blesser le malade par une surface aiguë.

Les deux coquilles entrent en contact l'une avec l'autre par leur lèvre débordante (8) prolongeant perpendiculairement les côtés latéraux (6) et parallélement au plan moyen des coquilles.

Les lèvres (8) était en contact, les deux coquilles sont maintenues en position fermée par un moins un moyen d'encliquetage formé par u ;ne partie mâle et une partie femelle complémentaire chacune se trouvant respectivement sur le coté latéral (6) de chaque coquille. La partie femelle se présente sous forme d'un renfoncement (26) vers l'intérieur de la coquille (2), de préférence conique, d'axe perpendiculaire au plan de la coquille et se rétrécissant en approchant de la lèvre. Elle se prolonge en dessous du plan de la lèvre par une saillie (13) qui se termine par une portion de cercle (14) d'une configuration sensiblement égale à la moitié d'un cercle et tourné vers l'extérieur.

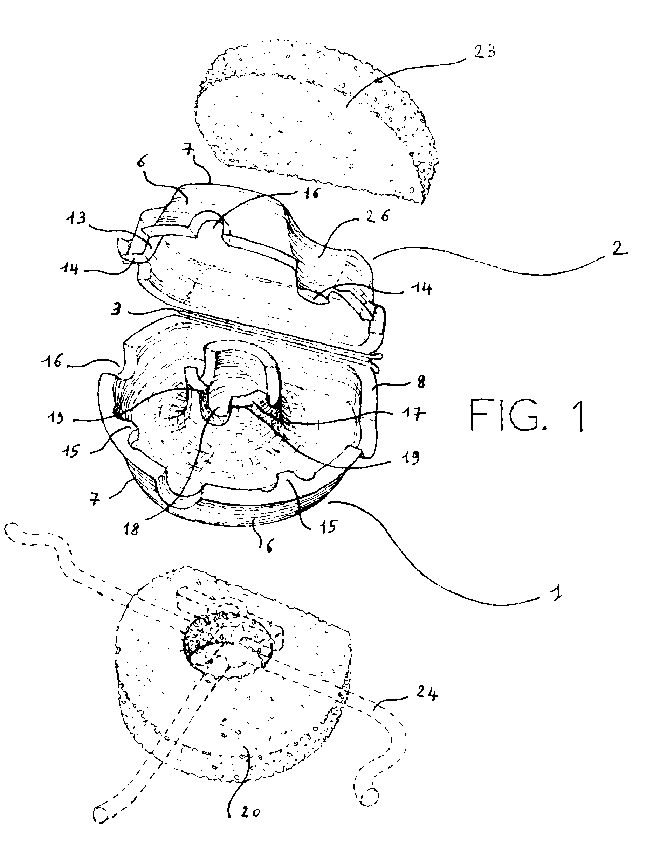

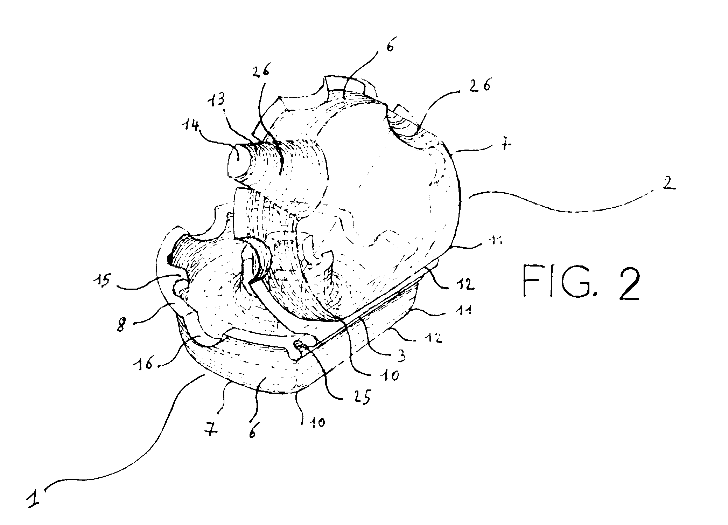

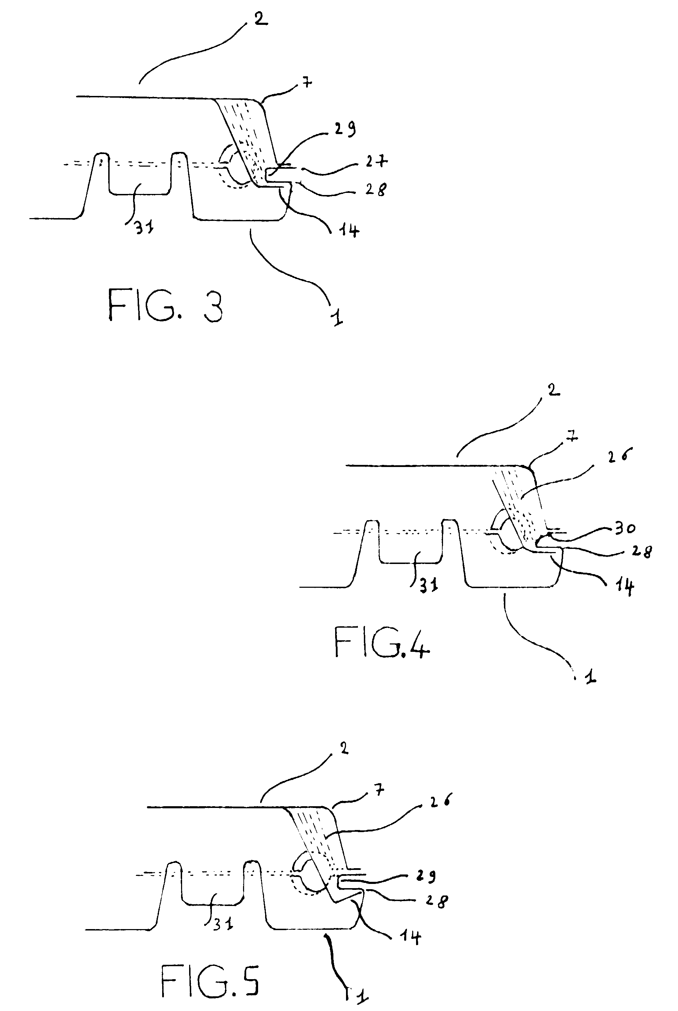

Vu de l'extérieur, la partie mâle a la forme d'une languette creuse (15) (fig. 1, fig.2). Elle est en relief vers l'intérieur de la coquille (fig.3) et dont le plateau supérieur (27) est le prolongement de la lèvre (8). Le plateau inférieur ( 28) se situe en dessous de la lèvre (8). Lorsque les deux coquilles (1,2) sont fermées, la face supérieure de la portion de cercle (14) se bloque sous le plateau inférieur (28) de la languette (15).

La portion de cercle (14) peut être avantageusement inclinée en faisant un angle aigu avec le plan de la coquille (2) de façon à ce que en rapprochant les deux coquilles (1,2), la face inférieure de la portion de cercle (14), glisse sur le plateau supérieur (27) de la languette (15)et se déplace radialement vers l'intérieur de la coquille, glisse le long du coté vertical (29) de la languette (15) puis vient reprendre sa position naturelle en se logeant sous le plateau inférieur (28) de la languette (fig. 5).

De même, le plateau supérieur de la languette (15) peut être incliné en descendant vers l'intérieur de la coquille, ceci permet à la portion de cercle (14) en appuie sur la partie inclinée ( 30 ) de la languette de glisser vers l'intérieur de l'étui puis de venir reprendre sa position naturelle en passant sous le plateau inférieur ( 28) de la languette (15) (fig. 4 ).

Les parties mâles ou femelles se montent indifféremment sur l'une ou l'autre coquilles (1,2). Avantageusement, la languette (15) équipe la coquille (1) et la saille (13) avec sa portion de cercle (14) équipe la coquille ( 2 ).

Le renfoncement conique (26) est suffisamment large au niveau supérieur pour recevoir le bout du doigt du manipulateur qui exerçant une pression pousse vers l'intérieur la portion de cercle (14) la dégageant du plateau ( 28 ) de la languette (15) pour ouvrir l'étui.

Un ou plusieurs moyens d'encliquetage peuvent équiper l'étui. Dans le cas d'un moyen unique, celui ci se place sur la partie centrale du bord latéral opposé à la charnière ( 3). S'il y a deux systèmes de fermeture, ils sont à égale distance des extrémités (10,11) de la portion de droite tronquée (12) et répartis à égales distance des sorties (16) des tubulures (24). Cette configuration est particulièrement intéressante car le pouce et l'index permettent d'ouvrir et fermer l'étui très facilement.

De préférence, sur le côté latéral ( 6 ) et sur la lèvre ( 8 ) un ou plusieurs orifices ( 16 ) sont pratiqués sensiblement pour moitié sur chaque coquille ( 1, 2 ) afin de laisser le passage aux tubulures ou autres tuyaux raccordés à l'article protégé entre les deux lits de mousse. Selon un exemple de réalisation, l'étui comprend trois orifices de sortie occupant les extrémités d'un «T» majuscule et de deux moyens d'encliquetage placés entre deux orifices.

Pour avoir un bon maintien de l'article sur la base (1) de l'étui, celle ci comporte avantageusement sur sa face intérieure un cylindre de retenu (17) sensiblement cylindrique s'élevant au centre de la base (1) et comportant un puits central (31). La périphérie verticale de ce puits comporte une ou plusieurs échancrures débouchantes (18) dont leur largeur au sommet est légèrement inférieure au diamètre des tubulures de raccord. Ce léger rétrécissement latéral ( 19) permet de clipser dans l'étui, avec une grande sécurité tous les types d'articles existant par l'intermédiaire de leurs tuyaux de sortie. Le diamètre du puits est égal ou supérieure au diamètre de la partie inférieure de l'article à protéger.

le diamètre intérieur du puits est compris entre 10 et 20 millimètres mais de préférence, il est de 14,5 millimètres et son épaisseur est comprise entre 1 et 5 millimètres mais de préférence 3 millimètres, la profondeur est comprise entre 8 et 16 millimètres mais de préférence elle est de 12 millimètres. Avantageusement, il n'y a plus de risque d'éjection du robinet lors de l'ouverture du boîtier par conséquent cela diminue d'autant le risque d'infections dues à la remise en place du robinet avec les doigts comme c'est le cas dans les autres types de boîtiers.

Le cylindre ( 17) est plus haut que l'épaisseur de la base (1) , cette différence est comprise entre 1 et 6 millimètres mais de préférence elle est de 4 millimètres. Ceci permet lors de l'ouverture du boîtier d'avoir un accès très aisé à la commande du robinet. Cette surélévation permet aussi de ne pas toucher d'autres parties du boîtier limitant encore les risques de contamination.

A l'intérieur de chaque coquille (1-2 ) une plaque de mousse est collée. La plaque de mousse dite de base ( 20 ) est percée au diamètre du cylindre. La plaque de mousse dite de couvercle ( 23 ) et celle dite de base ((20 ) est de type polyuréthanne, exempte de CFC dans sa fabrication, alvéolée par moitié ouverte et par moitié fermée afin de retenir un antiseptique l'imbibant.

Pour venir épouser très étroitement le robinet et les tubulures il est nécessaire que la mousse soit souple et sans aucune réservation ou échancrure et d'une épaisseur supérieure aux coquilles, de préférence, cette épaisseur est de 18 millimètres pour chaque mousse.

Cette fermeture dite intérieure assure de façon surprenante une meilleure hygiène d'utilisation car il n'y a plus aucun contact avec les doigts pour les opérations d'ouverture ou de fermeture comme c'est le cas avec les systèmes de fermetures dits extérieurs. Les languettes et les saillies sont protégées d'un toucher avec les doigts . Le cylindre ( 17 ) assure un excellent maintient de n'importe quel robinet et sa surélévation augmente l'hygiène lors des opérations de manipulations.

L'étui selon l'invention est très léger, moins de 5 grammes, pour une épaisseur de 23 millimètres, d'une largeur en son centre maximum de 55 millimètres et d'une longueur maximum de 65 millimètres. Il est parfaitement adapté en pédiatrie, chirurgie, urologie, réanimation et particulièrement en perfusion et nutrition parentérale. Les risques d'ouverture par déformation ou compression notamment en contact avec le corps sont pratiquement nuls car la liaison saillie languette est très sûre.

Selon une autre variante, l'étui peut avoir d'autres formes géométriques : carrée, rectangulaire et allant du pentagone au décagone pour l'utilisation d'une rampe ou d'un robinet ayant davantage de tubulures.

## Revendications

1. Etui de protection pour article médical, notamment pour un robinet à usage médical pour condition aseptique pour matériel médical, instrument de régulation ou autres, constitué d'au moins deux coquilles ( 1-2 ) reliées et articulées l'une par rapport à l'autre par une charnière ( 3 ) caractérisé en ce qu'il est constitué d'un matériau semi rigide thermoformé et en ce qu'il comporte au moins un moyen de fermeture des coquilles par encliquetage.

2. Etui de protection selon la revendication 1, caractérisé en ce que les moyens d'encliquetage comprennent une languette creuse (15) porté par le coté latéral (6) d'une coquille, coopérant avec une portions de cercle (14) portée par une saillie (13) se prolongeant par un renfoncement conique (26) porté par le coté latéral (6) de l'autre coquille.

3. Etui selon l'une des revendications 1 ou 2, caractérisé en ce que les moyens d'encliquetage sont obtenus par déformation vers l'intérieur de la paroi latéral ( 6 ) avant d'une coquille de façon à ce que la portion de cercle ( 14 ) de chaque saillie (13) vienne se loger sous le plateau inférieur( ( 28 ).de l'autre coquille.

4. Etui selon l'une quelconque des revendications précédentes caractérisé en ce que la charnière est en forme de deux portions de cercles reliées entre elles par une pliure en forme de « U » ouvert ( 25 ) vers l'extérieur de façon à ce que le boîtier ouvert, sans contrainte, les coquilles fassent entre elles un angle allant de 60° à 150°.

5. Etui selon l'une des revendications précédentes caractérisé en ce que les cotés latéraux ( 6 ) des coquilles ( 1, 2 ) comportent un ou des orifices ( 16 ) permettant le passage des tubulures ( 24 ).

6. Etui selon l'une des revendications précédentes caractérisé en qu'il comporte sur l'une des coquilles (1) un système de retenue protégé comportant des échancrures (18) qui présentent dans leur partie supérieure un léger rétrécissement latéral (19) maintenant les tubulures de raccordement de l'article protégé pour permettre de le retenir sur la dite coquille (1).

7. Etui selon la revendication 6 caractérisé en ce que le système de retenue est constitué d'un cylindre en relief(17) solidaire de la base (1) dont la partie haute dépasse le plan des lèvres de chaque coquille et qui comporte en son centre un puits ( 30 ) dont le fond est placé dans un plan plus élevé que le plan du fond de la base (1).

8. Etui selon l'une des revendications précédentes caractérisé en ce qu'il est themoformé en une seule pièce en un matériau ayant les mêmes propriétés de souplesse, rigidité et élasticité qu'un PVC de type cristal sous une épaisseur sensiblement constante de l'ordre de 0,2 à 1 millimètre d'épaisseur.

9. Etui selon l'une des revendications précédentes caractérisé en ce que la mousse ( 20, 23 ) des coquilles ( 1, 2 ) est en polyuréthanne dont les cellules la constituant sont pour moitié ouvertes et pour moitié fermées.

10. Etui selon l'une des revendications précédentes, caractérisé en ce qu'il est en P.V.C. du type « cristal « d'une épaisseur de 0,4 millimètre.
